# EUROPEAN PATENT APPLICATION

(11) **EP 2 544 130 A1**
(43) Date of publication of application: **09.01.2013**
(21) Application number: 11172896.0
(22) Date of filing: 06.07.2011
(51) Int. Cl.: G06Q 10/00

(54) **Digital patient agenda**

(71) Applicant: Koninklijke Philips Electronics N.V., 5621 BA Eindhoven (NL)
(72) Inventor: Van De Sluis, Bartel, M., 5600 AE Eindhoven (NL); Cuppen, Roel, P., 5600 AE Eindhoven (NL); Van Loenen, Evert, J., 5600 AE Eindhoven (NL); Brouns, Robin, C., A., 5600 AE Eindhoven (NL)
(74) Representative: Damen, Daniel Martijn

(57) **Abstract**

The invention relates to a system (102) for presenting a visual overview of scheduled activities to a patient. The system (102) comprises a receiver (104) for receiving a data link to a database for retrieving said scheduled activities, and an electronic display (106) for displaying along a timeline (108) visual elements (110, 112, 114, 116) representing said scheduled activities. The (102) system effectively reduces a patient's stress caused by ignorance regarding the activities scheduled for the coming hours and days.

## Description

### FIELD OF THE INVENTION

The invention relates to a system for presenting a visual overview of scheduled activities to a patient.

### BACKGROUND OF THE INVENTION

Patients in hospital environments often experience stress because of their health status and uncertainty about their future in connection therewith. For many patients, those feelings of stress are increased by a lack of information regarding the activities scheduled for the coming hours and days.

### SUMMARY OF THE INVENTION

It is an object of the invention to provide a system for presenting a visual overview of scheduled activities to a patient capable of effectively reducing a patient's stress level. This object is achieved by the system according to the invention, which system comprises a receiver for receiving a data link to a database for retrieving said scheduled activities, and an electronic display for displaying along a timeline a visual element representing a scheduled activity.

The system according to the invention enables a patient to gain upfront information relating to activities scheduled for him or her in a most convenient way via the electronic display. That is, the electronic display is configured for depicting timeline which adheres to the concept of an agenda known by intuition and for displaying a visual element which is easily accessible per se. As a result the system removes ignorance at the patient regarding those scheduled activities without creating any further stress caused by operation of the system itself. Therefore, the system is capable of effectively reducing a patient's stress level. By way of example, the system prevents from a patient being surprised by a nurse just before a therapy or medical intervention is about to take place.

In this text, scheduled activities include, without limitation, therapy, medical interventions, doctor's visits, relatives' visits and meals.

More and more, patients and their relatives take the liberty to ask nurses or other medical staff questions regarding e.g. therapies and interventions scheduled for today and the coming days. Answering this kind of questions can be very time-consuming as nurses usually do not know for each and every patient the scheduled activities and hence need to consult a colleague or a database before being able to give feasible answers. Therefore, the system according to the invention has the additional advantage of preventing from time-consuming interruptions of the tightly scheduled workflow of medical staff.

Owing to the electronic display, the system according to the invention furthermore has the additional advantage of being particularly suited for patient suffering from (i) aphasia i.e. the loss of ability to speak, listen, read or write, (ii) visual impairments, and (iii) cognitive disabilities.

A preferred embodiment of the system according to the invention comprises a camera for generating video recordings. This embodiment enables a patient to repeat the vast amount information distributed by medical staff during a scheduled activity such as a therapy session. Hence, during such session the patient is not continually occupied with the fear that he will not remember all instructions provided. As a result this embodiment is advantageous in that it further reduces a patient's stress level.

A further preferred embodiment of the system according to the invention comprises a microphone for generating audio recordings. This embodiment enables a patient to repeat the vast amount information distributed by medical staff during a scheduled activity such as a therapy session. Hence, during such session the patient is not continually occupied with the fear that he will not remember all instructions provided. As a result this embodiment is advantageous in that it further reduces a patient's stress level.

In a practical embodiment of the system according to the invention, the system is configured for starting the generation of the audio and video recordings based on a scheduled start time of the scheduled activity. This embodiment advantageously guarantees that the audio and video recordings register the scheduled activity in full.

A further preferred embodiment of the system according to the invention comprises a connection to the internet for streaming to a remote person the audio and video recordings. This embodiment enables relatives, e.g. parents of an infant, to remotely attend a scheduled activity such as a therapy session. This embodiment enables relatives to directly receive information provided during such therapy session and circumvents the need for those relatives to retrieve such information from medical staff at a later instance. Therefore, this embodiment has the advantage of preventing from time-consuming interruptions of the tightly scheduled workflow of medical staff.

In a further preferred embodiment of the system according to the invention, the system is configured for starting the streaming of the audio and video recordings based on the scheduled start time of the scheduled activity. This embodiment advantageously guarantees that the remote person is involved during the entire scheduled activity.

A further preferred embodiment of the system according to the invention comprises a sensor infrastructure configured for recognizing an electronic tag, wherein the system is configured for adjusting the scheduled start time and/or a scheduled end time of the scheduled activity based on recognition of said electronic tag. This embodiment enables updating the overview of scheduled activities based on the actual time of arrival at the patient by medical staff equipped with the electronic tag. Likewise, this embodiment enables updating the overview of scheduled activities based on the actual time of departure by said medical staff. Therefore, this embodiment has the advantage of providing a more up-to-date hence more accurate overview of the scheduled activities.

In a further preferred embodiment of the system according to the invention, the system is configured for starting the generation of audio and video recordings based on the recognition of the electronic tag. This embodiment advantageously guarantees that the audio and video recordings register the scheduled activity in full.

In a further preferred embodiment of the system according to the invention, the system is configured for starting the streaming of audio and video recordings based on recognition of the electronic tag. This embodiment advantageously guarantees that the remote person is involved during the entire scheduled activity.

In a further preferred embodiment of the system according to the invention, wherein the system is configured for moving the visual element and the timeline along the electronic display indicative for the time evolving. As a result, a visual element representing a past scheduled activity disappears from the electronic display whereas a visual element representing a future scheduled activity appears on the electronic display. In this embodiment the system represents only those scheduled activities that are within a limited time span, thereby improving the legibility of the display. As a result, this embodiment is advantageous in that it reduces a patient's stress level associated with the operation of the system.

In a further preferred embodiment of the system according to the invention, the system is configured for making a length of the visual element along the timeline indicative for the duration of the scheduled activity represented by said visual element. In this embodiment the system displays the scheduled activity in an even more intuitive fashion thereby further improving the legibility of the display. As a result, this embodiment advantageously further reduces the patient's stress level associated with the operation of the system.

In a further preferred embodiment of the system according to the invention, the system is configured for representing the scheduled start time of the scheduled activity by a leading edge of the visual element along the timeline. This embodiment prevents from explicitly indicating the scheduled start time in an alphanumeric fashion and hence prevents from raising expectations which may be hard to deliver in a hospital setting. Not delivering on these expectations may be a source of stress for the patient at hand. Consequently this embodiment has the advantage in that it prevents form further patient stress coming into being.

In a further preferred embodiment of the system according to the invention, the system is configured for representing the scheduled end time of the scheduled activity by a trailing edge of the visual element along the timeline. This embodiment prevents from explicitly indicating the scheduled end time in an alphanumeric fashion and hence prevents from raising expectations which may be hard to deliver in a hospital setting. Not delivering on these expectations may be a source of stress for the patient at hand. Consequently this embodiment has the advantage in that it prevents form further patient stress coming into being.

In a further preferred embodiment of the system according to the invention, the electronic display is a touch screen. As a result the display allows for e.g. scrolling along the timeline by the patient thereby enabling the patient to gain information regarding activities in the far future. Hence this embodiment provides the patient with a stronger feeling of control, thereby advantageously reducing a patient's stress level.

### SHORT DESCRIPTION OF THE FIGURES

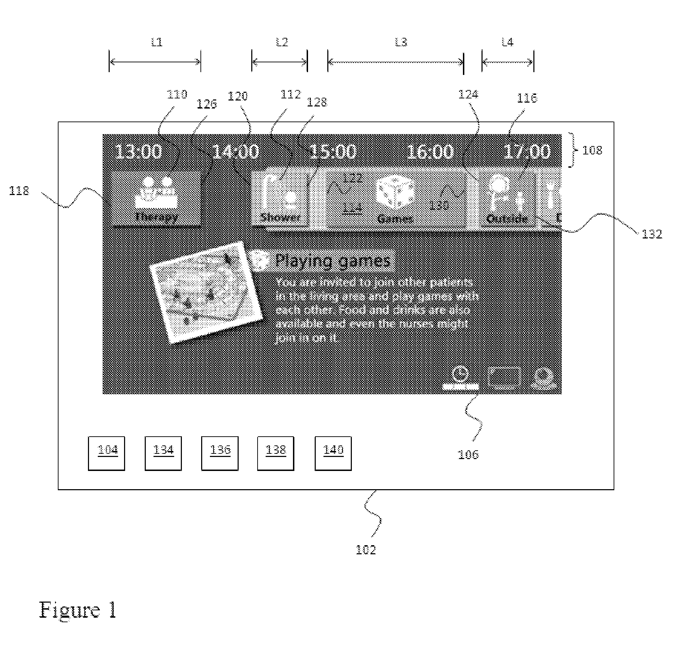

Fig. 1 schematically depicts an embodiment of the system according to the invention.

### DETAILED DESCRIPTION OF THE EMBODIMENTS

Figure 1 schematically displays a system 102 for presenting a visual overview of scheduled activities to a patient. The system 102 comprises a receiver 104 for receiving a data link to a database for retrieving said scheduled activities. The connection 104 is known per se such as a data link. The data link may bring about either simplex, half duplex or duplex communication. The system 102 furthermore comprises an electronic display 106 for displaying along a timeline 108 visual elements 110, 112, 114 and 116 representing scheduled activities. In this specific example, said scheduled activities concern therapy, showering, jointly relaxing and walking outside, respectively.

In this specific example the system 102 is configured for moving the visual elements 110, 112, 114 and 116 and the timeline 108 along the electronic display 106 indicative for the time evolving. In this particular example such movement occurs in a direction towards the left. In this particular embodiment the system 102 is configured for making the lengths L1, L2, L3 and L4 of the visual elements 110, 112, 114 and 116 along the timeline 108 indicative for the duration of the scheduled activity represented by said visual elements. For instance, L2 exceeds L1 indicating the therapy session will take more time than the showering. In this specific example the system 102 is configured for representing along the timeline 108 the scheduled start times of the scheduled activities by leading edges 118, 120, 122, 124 associated with visual elements 110, 112, 114 and 116, respectively. Furthermore, in this particular example the system 102 is configured for representing along the timeline 108 the scheduled end times of the scheduled activities by trailing edges 126, 128, 130 and 132 associated with visual elements 110, 112, 114 and 116, respectively. For example, as indicated by leading edge 120 and trailing edge 128, showering is scheduled to start somewhere just after 14:00 hrs and to end just before 15:00 hrs, respectively. In this specific embodiment the electronic display 106 is a touch screen. For example, this allows the patient to retrieve more detailed information regarding jointly relaxing by pressing visual element 114.

In this specific embodiment the system 102 comprises a camera 134, e.g. a digital camera known per se, for generating video recordings. Furthermore, the system 102 in this specific example comprises a microphone 136 for generating audio recordings. The system 102 may be configured for starting the generation of said video and audio recordings based on a scheduled start time of a scheduled activity. In this particular example the system 102 furthermore comprises a connection 138 to the internet for streaming to a remote person the audio and video recordings. The system 102 may be configured for starting said streaming based on the scheduled start time of a scheduled activity.

The system 102 in this particular example comprises a sensor infrastructure 140 for recognizing an electronic tag, wherein the system is configured for adjusting the scheduled start time and/or a scheduled end time of the scheduled activity based on recognition of said electronic tag. The sensor infrastructure 140 may be an RFID reader known per se whereas the electronic may be an RFID badge. The system 102 may be configured for starting the generation of the video and audio recordings based on the recognition of said electronic tag. The system 102 may furthermore be configured for starting the streaming of audio and video recordings to a person remoter based on the recognition of the electronic tag.

While the invention has been illustrated and described in detail in the drawings and in the foregoing description, the illustrations and the description are to be considered illustrative or exemplary and not restrictive. The invention is not limited to the disclosed embodiments. It is noted that the system according to the invention and all its components can be made by applying processes and materials known per se. In the set of claims and the description the word "comprising" does not exclude other elements and the indefinite article "a" or "an" does not exclude a plurality. Any reference signs in the claims should not be construed as limiting the scope. It is further noted that all possible combinations of features as defined in the set of claims are part of the invention.

## Claims

1. A system (102) for presenting a visual overview of scheduled activities to a patient, comprising:
a receiver (104) for receiving a data link to a database for retrieving said scheduled activities, and
an electronic display (106) for displaying along a timeline (108) a visual element (110, 112, 114, 1116) representing a scheduled activity.

2. The system according to claim 1, comprising a camera (134) for generating video recordings.

3. The system according to claim 2, comprising a microphone (136) for generating audio recordings.

4. The system according to claim 3, wherein the system is configured for starting the generation of the audio and video recordings based on a scheduled start time of the scheduled activity.

5. The system according to claim 3, comprising a connection (138) to the internet for streaming to a remote person the audio and video recordings.

6. The system, according to claim 5, wherein the system is configured for starting the streaming of the audio and video recordings based on the scheduled start time of the scheduled activity.

7. The system according to claim 1, further comprising a sensor infrastructure (140) configured for recognizing an electronic tag, wherein the system is configured for adjusting the scheduled start time and/or a scheduled end time of the scheduled activity based on recognition of said electronic tag.

8. The system according to claim 3 and claim 7, wherein the system is configured for starting the generation of audio and video recordings based on the recognition of the electronic tag.

9. The system according to claim 5 and claim 7, wherein the system is configured for starting the streaming of audio and video recordings based on recognition of the electronic tag.

10. The system according to claim 1, wherein the system is configured for moving the visual element (110, 112, 114, 1116) and the timeline (108) along the electronic display (106) indicative for the time evolving.

11. The system according to claim 1 wherein the system is configured for making a length (L1, L2, L3, L4) of the visual element (110, 112, 114, 1116) along the timeline (108) indicative for the duration of the scheduled activity represented by said visual element.

12. The system according to claim 1 wherein the system is configured for representing the scheduled start time of the scheduled activity by a leading edge of the visual element (110, 112, 114, 1116) along the timeline (108).

13. The system according to claim 1 wherein the system is configured for representing the scheduled end time of the scheduled activity by a trailing edge of the visual element (110, 112, 114, 1116) along the timeline (108).

14. The system according to claim 1 wherein the electronic display (106) is a touch screen.
